# FASCICULE DE BREVET EUROPEEN

(11) **EP 1 853 170 B1**
(45) Date de publication et mention de la délivrance du brevet: **28.09.2011**
(21) Numéro de dépôt: 05807545.8
(22) Date de dépôt: 28.09.2005
(51) Int. Cl.: A61B 8/08

(54) **INSTRUMENT DE MESURE DE L'ELASTICITE D'UN ORGANE COMPORTANT UN MOYEN DE CENTRAGE**
INSTRUMENT ZUR MESSUNG DER ORGANELASTIZITÄT MIT EINEM ZENTRIERMITTEL
INSTRUMENT FOR MEASURING ORGAN ELASTICITY COMPRISING A CENTRING MEANS

(30) Priorité: 28.09.2004 FR 0410265
(43) Date de publication de la demande: 14.11.2007
(73) Titulaire: Echosens, 75013 Paris (FR)
(72) Inventeur: SANDRIN, Laurent, F-92240 l'Hay-les-Roses (FR); HASQUENOPH, Jean-Michel, F-77860 Couilly-Pont-aux-Dames (FR)
(74) Mandataire: Lebkiri, Alexandre
(86) Numéro de dépôt international: PCT/FR2005/002397
(87) Numéro de publication internationale: WO 2006/035160

(56) Documents cités:
- FR-A- 2 843 290
- US-A- 4 881 549
- US-A1- 2004 006 266

## Description

La présente invention se rapporte au domaine de la mesure des propriétés viscoélastiques des tissus mous.

La présente invention se rapporte plus particulièrement à un instrument de mesure de l'élasticité d'un organe humain ou animal disposé derrière les côtes dudit humain ou animal.

Elle s'applique en particulier, mais non exclusivement, à la mesure de l'élasticité du foie d'un humain ou d'un animal, l'intérêt de cette mesure étant que cette dernière est corrélée à la quantité de fibrose présente dans le foie. En effet, les hépatites chroniques, pouvant être d'origine alcoolique, virale ou autre, présentent un effet fibrosant qu'il est important de connaître pour apprécier le meilleur moment pour traiter ces hépatites.

L'art antérieur connaît déjà de tels instruments de mesure.

Il est proposé en effet dans la demande de brevet internationale W02004/016176, déposée par le présent demandeur, un dispositif pour la mesure de l'élasticité d'un organe humain ou animal comprenant au moins un palpeur comportant un transducteur ultrasonore, au moins un capteur de position, un actionneur électrodynamique asservi, fixé au transducteur ultrasonore, apte à générer un coup basse fréquence transitoire. Le coup basse fréquence transmis par le transducteur ultrasonore entraîne la propagation dans les tissus d'une onde élastique dont la vitesse dépend de l'élasticité du milieu.

Cependant, un tel dispositif présente certains inconvénients, notamment lorsqu'il s'agit de mesurer l'élasticité d'organes placés derrière les côtes de l'humain ou de l'animal, comme le foie.

En effet, pour mesurer l'élasticité de ces organes, le dispositif est disposé contre la face avant de la cage thoracique, l'extrémité libre du transducteur étant placée entre deux côtes adjacentes (espace intercostal). Cependant, le positionnement du transducteur dans l'espace intercostal, mais également son maintien lorsque celui-ci est déplacé dans ce même espace intercostal, reste relativement peu aisé. Il résulte donc une certaine probabilité que l'extrémité du transducteur se positionne non pas dans un espace intercostal, mais directement sur une côte, générant alors des mesures non valides.

En outre, la perpendicularité de l'axe du transducteur par rapport à la surface de la peau, qui est nécessaire pour obtenir une bonne transmission des ondes élastique basse fréquence et ultrasonore, n'est pas toujours assurée.

Par ailleurs, afin que l'onde élastique basse fréquence émise par le transducteur ultrasonore vibrant passe au travers de la peau de l'humain ou de l'animal et atteindre l'organe à mesurer, il est nécessaire d'exercer une pression suffisante du dispositif contre celle-ci. Ceci est d'autant plus vrai pour les patients présentant une surcharge pondérale importante. Dans le dispositif susmentionné, la pression exercée est directement supportée par le transducteur et donc l'actionneur électrodynamique sur lequel il est fixé, ce qui peut générer une détérioration de la qualité de la vibration.

La présente invention entend remédier aux inconvénients de l'art antérieur en proposant un instrument de mesure permettant de guider le transducteur entre deux côtes adjacentes afin d'éviter que celui-ci ne soit directement en contact avec une desdites côtes.

La présente invention a également pour but de permettre le glissement de l'instrument de mesure le long de l'espace intercostal.

La présente invention a également pour but de soulager le geste du praticien, le poids de l'instrument de mesure étant partiellement appliqué sur l'humain ou l'animal.

Par ailleurs, la présente invention a pour objet d'offrir une transmission de la vibration améliorée en diminuant le phénomène de recul de l'instrument de mesure lors du choc lié à l'émission de l'onde élastique basse fréquence par le dispositif de mesure.

La présente invention a également pour objet de proposer un instrument de mesure permettant l'écartement de deux côtes adjacentes. Un tel instrument présentera donc un intérêt particulier pour les patients présentant un espace intercostal étroit.

La présente invention a également pour objet de proposer un instrument de mesure pouvant être adapté à la morphologie du patient.

La présente invention a également pour but de proposer un instrument de mesure qui diminue la pression supportée par l'actionneur électrodynamique.

La présente invention a également pour but d'améliorer la perpendicularité de l'axe du transducteur par rapport à la surface de la peau.

Pour ce faire, la présente invention concerne un instrument de mesure de l'élasticité d'un organe humain ou animal disposé derrière les côtes dudit humain ou animal, ledit instrument de mesure étant constitué d'un boîtier comprenant un moyen pour générer une onde élastique basse fréquence et un transducteur prévu pour être actionné de sorte à mesurer l'élasticité dudit organe. Elle est remarquable, dans son acception la plus large, en ce que l'instrument de mesure comprend en outre un moyen de centrage du transducteur entre les côtes.

De préférence, le moyen de centrage comprend au moins deux butées prévues de part et d'autre du transducteur.

Avantageusement, lesdites butées présentent des dimensions transversales permettant la mise en appui desdites butées contre des côtes.

Avantageusement, lesdites butées présentent une forme arrondie.

Selon un mode de réalisation préféré de l'invention, le moyen de centrage est formé d'une bague fixée au boîtier, ladite bague comprenant un alésage dans lequel ledit transducteur est disposé.

Avantageusement, la bague consiste en une couronne annulaire sur laquelle lesdites butées sont disposées de manière diamétralement opposée.

Avantageusement, la bague est fixée au boîtier par l'intermédiaire de ressorts.

Avantageusement, la bague est amovible.

Selon une variante de l'invention, le moyen de centrage est constitué par un ou plusieurs capteurs de pression.

Selon une autre variante de l'invention, le moyen de centrage est constitué par un ou plusieurs transducteurs ultrasonores.

Selon une autre variante particulièrement avantageuse de l'invention, le moyen de centrage comporte au moins un transducteur d'imagerie permettant la formation d'une image échographique dudit organe. Plus particulièrement, au moins une des butées formant le moyen de centrage est constituée par une paroi transparente aux ultrasons, ladite paroi délimitant une cavité dans laquelle est logé le transducteur d'imagerie.

L'opérateur peut ainsi, lors de la mesure de l'élasticité de l'organe étudié, se repérer dans cet organe par l'affichage d'une image échographique correspondante.

De préférence, le transducteur d'imagerie est un transducteur de type monoélément. Pour permettre la formation d'images échographiques, un tel transducteur est alors monté articulé sur ledit instrument selon un axe de pivotement transversal.

Avantageusement, ledit transducteur est motorisé.

Afin de faciliter le passage des ultrasons du transducteur d'imagerie en direction de la paroi de la butée associée, la cavité est remplie d'un liquide de couplage. Avantageusement, la cavité comprend une membrane d'étanchéité destinée à empêcher que le liquide ne s'écoule dans les parties électroniques de l'instrument de mesure (moteur commandant les transducteurs, ...). Le liquide de couplage est ainsi retenu par la membrane d'étanchéité dans la cavité. Avantageusement, la membrane d'étanchéité est une membrane souple afin de permettre le pivotement du transducteur d'imagerie dans la cavité.

Selon un mode de réalisation particulier de l'invention, l'instrument de mesure comporte en outre une membrane de protection recouvrant l'extrémité distale dudit instrument de mesure, laquelle extrémité distale est formée par le transducteur et le moyen de centrage.

Avantageusement, le moyen pour générer l'onde élastique basse fréquence est formé par le transducteur ou le moyen de centrage.

De même, dans un mode de réalisation avantageux de l'invention, l'instrument de mesure comprend un dispositif de chauffage permettant de chauffer ledit moyen de centrage. Ainsi chauffée par l'intermédiaire du moyen de centrage, la peau offre une meilleure propagation des ondes ultrasonores.

On comprendra mieux l'invention à l'aide de la description, faite ci-après à titre purement explicatif, en référence aux figures annexées :
- la figure 1 illustre une vue en perspective d'un instrument de mesure selon un premier mode de réalisation de l'invention ;
- la figure 2 illustre une vue latérale partielle en coupe de l'instrument de mesure de la figure 1 ;
- la figure 3 illustre une vue en perspective du transducteur utilisé avec l'instrument de mesure de la figure 1 ;
- la figure 4 illustre une vue en perspective du moyen de centrage dudit instrument de mesure de la figure 1 ; et
- la figure 5 illustre une vue en perspective partielle d'un instrument de mesure selon un second mode de réalisation de l'invention ;
- la figure 6 illustre une vue détaillée d'un moyen de centrage d'un instrument de mesure selon un mode de réalisation particulier de l'invention ;
- la figure 7 schématise le fonctionnement d'un instrument de mesure équipé du moyen de centrage illustré sur la figure 6.

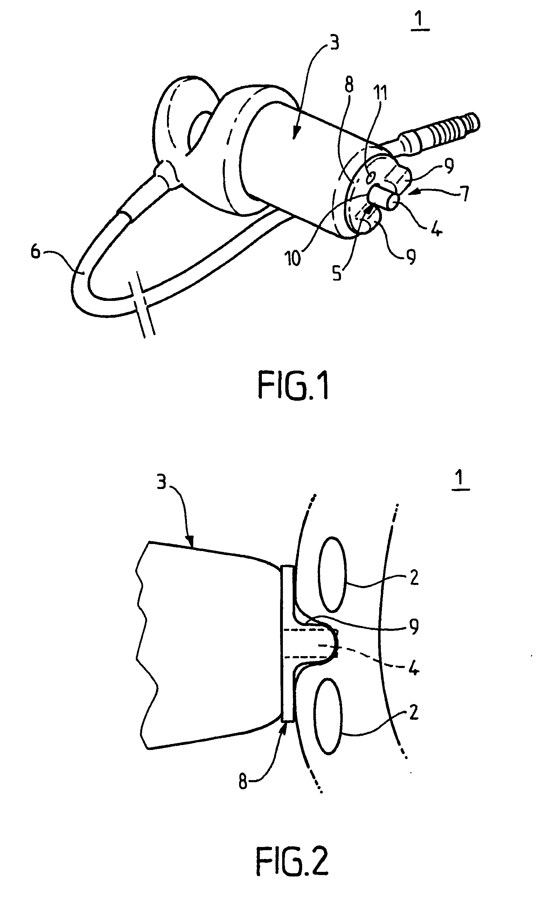

La figure 1 illustre un instrument de mesure (1) de l'élasticité d'un organe humain ou animal selon un premier mode de réalisation de l'invention.

Ledit instrument de mesure (1) est destiné en particulier à mesurer l'élasticité d'un organe humain ou animal disposé derrière les côtes (2) dudit humain ou animal.

Ledit instrument de mesure (1) présente un boîtier (3) dans lequel est logé un actionneur électrodynamique (non représenté). A l'extrémité de l'actionneur est monté un transducteur (4) ultrasonore mobile dont une partie de l'extrémité distale (5) s'étend hors du corps (3) dudit l'instrument de mesure (1). L'instrument de mesure (1) est en outre relié à une source d'énergie par liaison filaire (6).

L'invention ayant pour objet un perfectionnement d'un instrument de mesure déjà existant (instrument de mesure décrit dans la demande de brevet W02004/016176), nous ne nous attacherons pas à décrire plus en détail sa structure. Pour la mise en oeuvre dudit instrument de mesure (1), ainsi que pour la compréhension de son fonctionnement, l'homme du métier se référera à la demande de brevet internationale W02004/016176. Cependant, il est bien entendu évident qu'il s'agit ici d'une configuration d'instrument de mesure donné à titre explicatif. D'autres configurations pourront être envisagées par un homme du métier sans sortir du cadre de l'invention. En particulier, il pourra être envisagé un instrument de mesure ne comportant pas d'actionneur, la fonction dudit actionneur étant prise en charge par exemple directement par ledit transducteur.

Afin de permettre le positionnement et le maintien de l'extrémité distale (5) du transducteur (4) entre deux côtes adjacentes, ledit instrument de mesure (1) comporte un moyen de centrage (7) du transducteur (4) entre les côtes.

Le moyen de centrage (7), illustré sur la figure 1, consiste avantageusement en une bague (8) fixée au boîtier (3), la face libre de ladite bague (8) présentant deux butées (9). Ladite bague (7) présente un alésage (10) de sorte que, lorsque la bague est fixée audit boîtier (3), l'extrémité distale (5) dudit transducteur (4) traverse l'alésage (10) formée dans ladite bague (8).

Dans l'exemple de réalisation illustré sur la figure 1, la bague (8) est fixée audit instrument de mesure (1) au moyen de vis (11). Il est bien entendu évident que la présente invention ne se limite pas à ce mode de fixation. La bague (8) pourra, par exemple, être configurée pour venir se clipser, de préférence de manière réversible, sur ledit instrument de mesure (1).

Mais dans tous les cas, ladite bague (8) sera de préférence amovible. Ainsi, selon la morphologie du patient, il sera procédé à des échanges de bague (8) afin d'obtenir un instrument de mesure (1) dont les moyens de centrage (7) sont adaptés à l'espace intercostal du patient examiné (adaptation par exemple des butées en terme de hauteur ou de dimensions transversales).

Avantageusement, l'alésage (10) est disposé au centre de ladite bague (8).

Avantageusement, les deux butées (9) sont disposées de manière diamétralement opposée de part et d'autre de l'alésage (10) lequel est traversé par l'extrémité distale (5) du transducteur (4).

Avantageusement, lesdites butées (9) constituent des capteurs de pression ou des transducteurs ultrasonores.

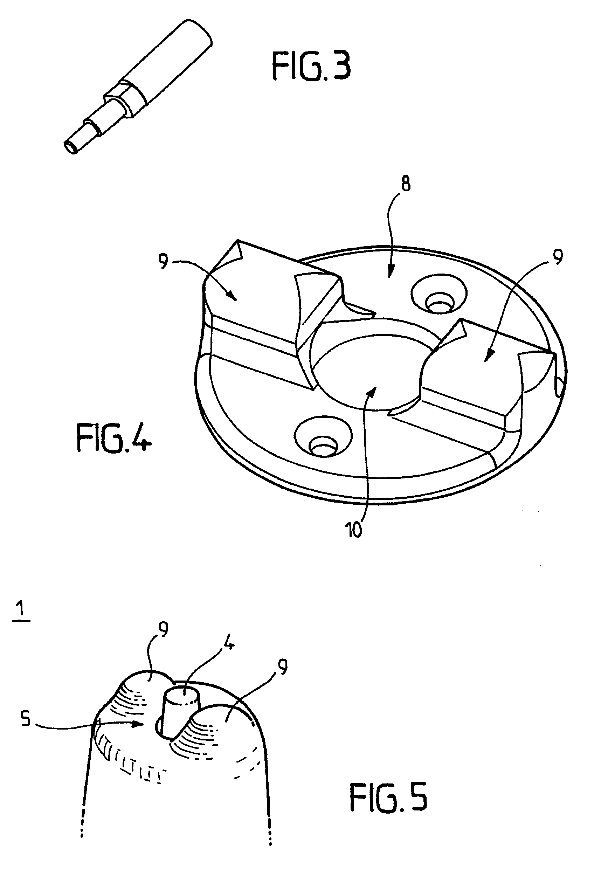

La figure 4 illustre plus clairement la forme et la structure d'une telle bague.

La figure 2 illustre une vue partielle latérale de l'instrument de mesure (1) lorsque celui-ci est positionné contre la cage thoracique d'un patient.

Plus particulièrement, lorsque l'instrument de mesure (1) est placé contre la cage thoracique, les butées (9) de la bague (8) sont telles qu'elles se positionnent dans l'espace (11) formé entre deux côtes (2) adjacentes, disposant en conséquence l'extrémité distale (5) du transducteur (4) dans l'espace intercostal (11).

Comme expliqué plus haut, il est nécessaire, pour permettre la mesure de l'élasticité d'un organe, de maintenir l'instrument de mesure (1) contre la cage thoracique avec une certaine pression afin que les ondes soient correctement transmises au travers de la peau du patient. Les butées (9) étant destinées également à empêcher que cette pression ne soit supportée uniquement par le transducteur (4), la hauteur des butées (9) est définie pour que celles-ci soient au contact de la peau lorsque la pression exercée est suffisante pour permettre une transmission correcte des ondes basse fréquence au travers de la peau du patient, et donc pour permettre l'examen.

De plus, les butées (9) présentent avantageusement des dimensions transversales permettant leur mise en appui contre les côtes (2) entre lesquelles elles sont disposées.

Avantageusement, lesdites butées (9) présentent une forme arrondie de sorte que, lorsque l'instrument de mesure (1) est pressé contre la cage thoracique, celles-ci ne blessent pas le patient.

Afin de diminuer encore ce risque de blessure, les butées (9) seront constituées avantageusement d'un matériau déformable (caoutchouc, ...). Ainsi, non seulement la pression exercée sur la surface de la peau par lesdites butées (9) sera mieux répartie, mais également le choc lié à l'émission de l'onde élastique basse fréquence par le transducteur (4) amorti. De même, la bague (8) pourra être également fixée au boîtier (3) dudit instrument de mesure (1) par l'intermédiaire d'éléments formant ressort (non représentés).

Selon une configuration particulière de l'invention, l'instrument de mesure (1) comprend en outre une membrane de protection recouvrant la bague (8). Cette membrane a pour fonction non seulement de protéger l'instrument de mesure (1) de tout encrassement, notamment par l'application d'un gel favorisant la transmission des ondes, mais également d'éviter la contamination d'un patient à un autre par l'utilisation d'une nouvelle membrane à chaque nouvelle intervention sur un patient.

Avantageusement, ladite membrane comprend du gel échographique afin d'assurer un couplage ultrasonore correct.

Par ailleurs, afin d'éviter toute contamination d'un patient à un autre, ladite bague (8) est avantageusement jetable.

Concernant le transducteur (4) de l'instrument de mesure (1) illustré sur la figure 1, celui-ci est un transducteur du type circulaire. Il est présenté dans son ensemble sur la figure 3. Cette configuration du transducteur est donnée ici à titre d'exemple. Il est bien entendu évident que tout transducteur présentant une forme autre que circulaire pourra être utilisé.

La figure 5 illustre une vue en perspective partielle d'un instrument de mesure selon un second mode de réalisation de l'invention. Dans ce mode de réalisation, l'extrémité de l'instrument de mesure (1) destinée à être en contact avec la cage thoracique du patient présente deux butées (9) formant le moyen de centrage du transducteur (4) entre les côtes. Lesdites butées (9), comme dans le mode de réalisation de l'instrument de mesure précédemment décrit, sont disposées de part et d'autre de l'extrémité distale (5) du transducteur (4) s'étendant hors du boîtier (3).

De même que précédemment, l'instrument de mesure (1) est avantageusement équipé, à l'extrémité portant les butées (9), d'une membrane de protection protégeant le transducteur d'une éventuelle contamination lors de son contact avec la surface de la peau de l'humain ou de l'animal.

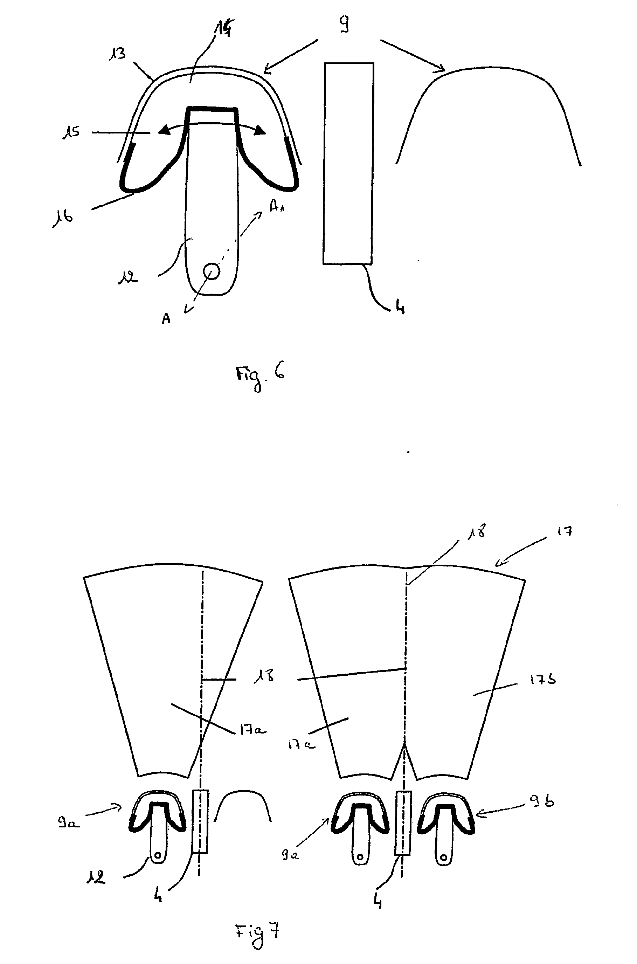

La figure 6 illustre une configuration particulièrement avantageuse des butées (9) formant le moyen de centrage (7) dudit instrument de mesure (1), lesdites butées (9) étant disposées de part et d'autre du transducteur (4) destiné à la mesure de l'élasticité.

Lesdites butées (9) sont respectivement constituées par une paroi (13) délimitant une cavité (14) dans laquelle est logé un transducteur d'imagerie (12).

De préférence, le transducteur d'imagerie (12) est un transducteur de type monoélément. Pour permettre la formation d'images échographiques, un tel transducteur est monté articulé sur ledit instrument (1) selon un axe de pivotement transversal AA1.

Avantageusement, ledit transducteur est motorisé.

Il est bien entendu évident que l'homme du métier pourra utiliser des transducteurs de type barrettes échographiques multiéléments. Cependant, l'utilisation de transducteurs monoéléments présente l'avantage d'un coût réduit de réalisation dudit instrument de mesure (1) du fait qu'une seule voie électronique d'acquisition associée à un multiplexeur est nécessaire pour piloter séquentiellement les transducteurs monoélement constituant l'instrument de mesure (1), à savoir le transducteur (4) destiné à la mesure de l'élasticité de l'organe étudié et le transducteur d'imagerie (12).

Ainsi l'opérateur peut, lors de la mesure de l'élasticité de l'organe étudié, se repérer dans cet organe par l'affichage d'une image échographique correspondante comme l'illustre la figure 7. Le transducteur (12a, 12b) de chacune des butées (9a, 9b) balaie respectivement une zone (17a, 17b) de l'organe telle la zone totale (17) balayée par lesdits transducteurs d'imagerie (12) couvre l'axe (18) de la mesure de l'élasticité effectuée par le transducteur (4).

Par ailleurs, afin de faciliter le passage des ultrasons du transducteur d'imagerie (12) en direction de la paroi (13) de la butée (9) associée, la cavité (14) est remplie d'un liquide de couplage (15). Les parties électroniques dudit instrument de mesure (1), et en particulier le moteur actionnant le pivotement du transducteur d'imagerie (12), sont alors protégées par la mise en place dans la cavité (14) d'une membrane d'étanchéité (16). Ladite membrane (16) est disposée dans la cavité (14) de telle sorte que le liquide de couplage (15) est retenu contre la paroi (13), et plus particulièrement contre la paroi supérieure de la butée (9).

L'invention est décrite dans ce qui précède à titre d'exemple. Il est entendu que l'homme du métier est à même de réaliser différentes variantes de l'invention sans pour autant sortir du cadre du brevet.

## Revendications

1. Instrument de mesure (1) de l'élasticité d'un organe humain ou animal disposé derrière les côtes (2) dudit humain ou animal, du type constitué d'un boîtier (3) comprenant un moyen pour générer une onde élastique basse fréquence et un transducteur (4) prévu pour être actionné de sorte à mesurer l'élasticité dudit organe, **caractérisé en ce que** l'instrument de mesure (1) comprend en outre un moyen de centrage (7) du transducteur (4) entre les côtes (2).

2. Instrument de mesure (1) selon la revendication 1, **caractérisé en ce que** le moyen de centrage (7) comprend au moins deux butées (9) prévues de part et d'autre du transducteur (4).

3. Instrument de mesure (1) selon la revendication 2, **caractérisé en ce que** lesdites butées (9) présentent des dimensions transversales permettant la mise en appui desdites butées (9) contre les côtes (2).

4. Instrument de mesure (1) selon la revendication 2 ou la revendication 3, **caractérisé en ce que** lesdites butées (9) présentent une forme arrondie.

5. Instrument de mesure (1) selon l'une quelconque des revendications 1 à 4, **caractérisé en ce que** le moyen de centrage (7) est formé d'une bague (8) fixée au boîtier (3), ladite bague (8) comprenant un alésage (10) dans lequel ledit transducteur (4) est disposé.

6. Instrument de mesure (1) selon la revendication précédente, **caractérisé en ce que** la bague (8) consiste en une couronne annulaire sur laquelle lesdites butées (9) sont disposées de manière diamétralement opposée.

7. Instrument de mesure (1) selon la revendication 5 ou la revendication 6, **caractérisé en ce que** la bague (8) est fixée au boîtier (3) par l'intermédiaire d'éléments formant ressort.

8. Instrument de mesure (1) selon l'une quelconque des revendications 5 à 7, **caractérisé en ce que** la bague (8) est amovible.

9. Instrument de mesure (1) selon l'une quelconque des revendications 1 à 8, **caractérisé en ce que** le moyen de centrage (7) est constitué par au moins un capteur de pression.

10. Instrument de mesure (1) selon l'une quelconque des revendications 1 à 9, **caractérisé en ce que** le moyen de centrage (7) est constitué par au moins un transducteur ultrasonore (4).

11. Instrument de mesure (1) selon l'une quelconque des revendications 1 à 8, **caractérisé en ce que** le moyen de centrage (7) comporte au moins un transducteur d'imagerie (12) permettant la formation d'une image échographique dudit organe.

12. Instrument de mesure (1) selon l'une quelconque des revendications 2 à 11, **caractérisé en ce qu'**au moins une des butées (9) comprend un transducteur d'imagerie (12), la butée (9) étant constituée par une paroi (13) transparente aux ultrasons délimitant une cavité (14) dans laquelle est logé le transducteur d'imagerie (12).

13. Instrument de mesure (1) selon la revendication 11 ou la revendication 12, **caractérisé en ce que** le transducteur d'imagerie (12), du type monoélément, est monté articulé sur ledit instrument (1) selon un axe de pivotement transversal AA1.

14. Instrument de mesure (1) selon l'une quelconque des revendications précédentes 11 à 13, **caractérisé en ce qu'**il comprend un moteur actionnant le pivotement dudit transducteur (12).

15. Instrument de mesure (1) selon l'une quelconque des revendications 12 à 14, **caractérisé en ce que** la cavité (14) contient un liquide de couplage (15), lequel est retenu dans la cavité (14) par une membrane d'étanchéité (16).

16. Instrument de mesure (1) selon l'une quelconque des revendications 1 à 15, **caractérisé en ce qu'**il comporte en outre une membrane de protection déformable recouvrant l'extrémité distale dudit instrument de mesure (1) formée par le transducteur (4) et le moyen de centrage (7).

17. Instrument de mesure (1) selon l'une quelconque des revendications 1 à 16, **caractérisé en ce que** le moyen pour générer l'onde élastique basse fréquence est formé par le transducteur (4).

18. Instrument de mesure (1) selon l'une quelconque des revendications 1 à 16, **caractérisé en ce que** le moyen pour générer l'onde élastique basse fréquence est formé par le moyen de centrage (7).

19. Instrument de mesure (1) selon l'une quelconque des revendications 1 à 18, **caractérisé en ce que** l'instrument de mesure comprend un dispositif de chauffage pour chauffer ledit moyen de centrage (7).

## Claims

1. An instrument for measuring (1) the elasticity of a human or animal organ disposed behind the ribs (2) of said human or animal, of the type constituted of a housing (3) comprising a means to generate a low-frequency elastic wave and a transducer (4) provided to be activated so as to measure the elasticity of said organ, **characterized in that** the measuring instrument (1) also comprises a means (7) for centering the transducer (4) between the ribs (2).

2. The measuring instrument (1) according to claim 1, **characterized in that** the centering means (7) comprises at least two stops (9) provided on both sides of the transducer (4).

3. The measuring instrument (1) according to claim 2, **characterized in that** said stops (9) present transverse dimensions enabling said stops (9) to press against the ribs (2).

4. The measuring instrument (1) according to claim 2 or claim 3, **characterized in that** said stops (9) present a rounded shape.

5. The measuring instrument (1) according to any one of claims 1 to 4, **characterized in that** the centering means (7) is formed of a ring (8) fixed to the housing (3), said ring (8) comprising a bore (10) in which said transducer (4) is positioned.

6. The measuring instrument (1) according to the previous claim, **characterized in that** the ring (8) consists of an annular crown on which said stops (9) are disposed in a diametrically opposed manner.

7. The measuring instrument (1) according to claim 5 or claim 6, **characterized in that** the ring (8) is fixed to the housing (3) through elements forming a spring.

8. The measuring instrument (1) according to any one of claims 5 to 7, **characterized in that** the ring (8) is removable.

9. The measuring instrument (1) according to any one of claims 1 to 8, **characterized in that** the centering means (7) is constituted of at least one pressure sensor.

10. The measuring instrument (1) according to any one of claims 1 to 9, **characterized in that** the centering means (7) is constituted of at least one ultrasound transducer (4).

11. The measuring instrument (1) according to any one of claims 1 to 8, **characterized in that** the centering means (7) comprise at least one imaging transducer (12) enabling the formation of an echographic image of said organ.

12. The measuring instrument (1) according to any one of claims 2 to 11, **characterized in that** at least one of the stops (9) comprises an imaging transducer (12), the stop (9) being constituted of a wall (13) that is transparent to ultrasound, defining a cavity (14) in which the imaging transducer (12) is housed.

13. The measuring instrument (1) according to claim 11 or claim 12, **characterized in that** the imaging transducer (12), of the single-element type, is hinged on said instrument (1) along a transverse pivotal axis AA1.

14. The measuring instrument (1) according to any one of the previous claims 11 to 13, **characterized in that** the instrument comprises a motor activating the pivoting of said transducer (12).

15. The measuring instrument (1) according to any one of claims 12 to 14, **characterized in that** the cavity (14) contains a coupling liquid (15), which is retained in the cavity (14) by a sealing membrane (16).

16. The measuring instrument (1) according to any one of claims 1 to 15, **characterized in that** the instrument also comprises a deformable protective membrane covering the distal end of said measuring instrument (1), formed by the transducer (4) and the centering means (7).

17. The measuring instrument (1) according to any one of claims 1 to 16, **characterized in that** the means to generate the low-frequency elastic wave is formed by the transducer (4).

18. The measuring instrument (1) according to any one of claims 1 to 16, **characterized in that** the means to generate the low-frequency elastic wave is formed by the centering means (7).

19. The measuring instrument (1) according to any one of claims 1 to 18, **characterized in that** the measuring instrument comprises a heating device to heat said centering means (7).

## Patentansprüche

1. Messgerät (1) der Elastizität eines menschlichen oder tierischen Organs, das hinter den Rippen (2) des Menschen oder Tiers angeordnet ist, des Typs, der ein Gehäuse (3) bildet, das ein Mittel zum Erzeugen einer elastischen Niederfrequenzwelle aufweist, und einen Messwandler (4), der vorgesehen ist, um derart betätigt zu werden, dass die Elastizität des Organs gemessen wird, **dadurch gekennzeichnet, dass** das Messgerät (1) ferner ein Mittel zum Zentrieren (7) des Messwandlers (4) zwischen den Rippen (2) aufweist.

2. Messgerät (1) nach Anspruch 1, **dadurch gekennzeichnet, dass** das Zentriermittel (7) mindestens zwei Anschläge (9) aufweist, die zu beiden Seiten des Messwandlers (4) vorgesehen sind.

3. Messgerät (1) nach Anspruch 2, **dadurch gekennzeichnet, dass** die Anschläge (9) Quermaße aufweisen, die das Anlegen der Anschläge (9) gegen die Rippen (2) erlauben.

4. Messgerät (1) nach Anspruch 2 oder Anspruch 3, **dadurch gekennzeichnet, dass** die Anschläge (9) eine gerundete Form aufweisen.

5. Messgerät (1) nach einem der Ansprüche 1 bis 4, **dadurch gekennzeichnet, dass** das Zentriermittel (7) aus einem Ring (8) besteht, der an dem Gehäuse (3) befestigt ist, wobei der Ring (8) eine Bohrung (10) aufweist, in der der Messwandler (4) angeordnet ist.

6. Messgerät (1) nach dem vorhergehenden Anspruch, **dadurch gekennzeichnet, dass** der Ring (8) aus einem ringförmigen Kranz besteht, auf dem die Anschläge (9) diametral entgegengesetzt angeordnet sind.

7. Messgerät (1) nach Anspruch 5 oder Anspruch 6, **dadurch gekennzeichnet, dass** der Ring (8) an dem Gehäuse (3) über Elemente, die eine Feder bilden, befestigt ist.

8. Messgerät (1) nach einem der Ansprüche 5 bis 7, **dadurch gekennzeichnet, dass** der Ring (8) abnehmbar ist.

9. Messgerät (1) nach einem der Ansprüche 1 bis 8, **dadurch gekennzeichnet, dass** das Zentriermittel (7) aus mindestens einem Druckfühler besteht.

10. Messgerät (1) nach einem der Ansprüche 1 bis 9, **dadurch gekennzeichnet, dass** das Zentriermittel (7) aus mindestens einem Ultraschall-Messwandler (4) besteht.

11. Messgerät (1) nach einem der Ansprüche 1 bis 8, **dadurch gekennzeichnet, dass** das Zentriermittel (7) mindestens einen Bildtechnik-Messwandler (12) aufweist, der das Bilden eines Ultraschallbilds des Organs erlaubt.

12. Messgerät (1) nach einem der Ansprüche 2 bis 11, **dadurch gekennzeichnet, dass** mindestens einer der Anschläge (9) einen Bild Technik-Messwandler (12) aufweist, wobei der Anschlag (9) aus einer für Ultraschall durchsichtigen Wand (13) besteht, die einen Hohlraum (14) abgrenzt, in dem der Bildtechnik-Messwandler (12) untergebracht ist.

13. Messgerät (1) nach Anspruch 11 oder Anspruch 12, **dadurch gekennzeichnet, dass** der Bild Technik-Messwandler (12) des Typs aus einem Element ist und gelenkig auf dem Messgerät (1) entlang einer Querschwenkachse AA1 installiert ist.

14. Messgerät (1) nach einem der vorhergehenden Ansprüche 11 bis 13, **dadurch gekennzeichnet, dass** es einen Motor aufweist, der das Schwenken des Messwandlers (12) betätigt.

15. Messgerät (1) nach einem der Ansprüche 12 bis 14, **dadurch gekennzeichnet, dass** der Hohlraum (14) eine Koppelflüssigkeit (15) aufweist, die in dem Hohlraum (14) durch eine Dichtmembran (16) zurückgehalten wird.

16. Messgerät (1) nach einem der Ansprüche 1 bis 15, **dadurch gekennzeichnet, dass** es ferner eine verformbare Schutzmembran aufweist, die das distale Ende des Messgerätes (1), das von dem Messwandler (4) und dem Zentriermittel (7) gebildet wird, abdeckt.

17. Messgerät (1) nach einem der Ansprüche 1 bis 16, **dadurch gekennzeichnet, dass** das Mittel zum Erzeugen der elastischen Niederfrequenzwelle aus dem Messwandler (4) besteht.

18. Messgerät (1) nach einem der Ansprüche 1 bis 16, **dadurch gekennzeichnet, dass** das Mittel zum Erzeugen der elastischen Niederfrequenzwelle aus dem Zentriermittel (7) besteht.

19. Messgerät (1) nach einem der Ansprüche 1 bis 18, **dadurch gekennzeichnet, dass** das Messgerät eine Heizvorrichtung zum Heizen des Zentriermittels (7) aufweist.
